# EUROPEAN PATENT SPECIFICATION

(11) **EP 0 356 332 B1**
(45) Date of publication and mention of the grant of the patent: **25.05.1994**
(21) Application number: 89402320.9
(22) Date of filing: 22.08.1989
(51) Int. Cl.: C07C 53/128, C07F 15/00

(54) **Platinum complexes of single isomer neoalkyl acids**
Platinkomplexe mit einzelnen Isomeren von Neoalkylsäuren
Complexes de platine avec isomères uniques d'acides néoalkyles

(30) Priority: 22.08.1988 US 234961
(43) Date of publication of application: 28.02.1990
(73) Proprietor: THE LIPOSOME COMPANY, INC., Princeton, NJ 08540 (US)
(72) Inventor: Tremblay, Paul A., Hamilton New Jersey 08619 (US); Pilkiewicz, Frank, West Windsor New Jersey 08512 (US); Cherian, Mathew, Princeton New Jersey 08540 (US); Portnoff, Joel, Richboro Pennsylvania 18954 (US); Lenk, Robert P., Lambertville New Jersey 08530 (US)
(74) Representative: Ahner, Francis

(56) References cited:
- EP-A- 0 108 489
- EP-A- 0 193 936
- WO-A-87/02364
- DE-A- 2 044 312
- DE-B- 2 621 526
- CHEMISCHE BERICHTE, vol. 116, 1983, pages323-347; T. WEISKE et al.: "Pseudo-einstufige C-C-Spaltungen von ionisierten Carbonsäuren. Radikaltypische Reaktionen in der Massenspektroskopie"
- BULLETIN OF THE CHEMICAL SOCIETY OF JAPAN, vol. 49, no. 11, 1976, pages 3296-3299, Tokyo, JP; y. souma et al.: "Carbonylation of olefins, alcohols, and saturated hydrocarbons using Cu(CO)n1 and Ag(CO)21 Catalysts in BF3-H2O"
- CHEMICAL ABSTRACTS, vol. 99, no. 22, 1983, page 94, column 1, abstract no. 177578q, Columbus, Ohio, US; DAINIPPON & INK CHEMICALS: "Polyester powder coating compositions", & JP-A-58 34869
- CHEMICAL ABSTRACTS, vol. 95, no. 12, 1981, page 193, column 2, abstract no. 101020y, Columbus, Ohio, US; V.I. SOKOLOVA et al.: "Frothing agent for flotation of nonferrous metal ores "
- CHEMICAL ABSTRACTS, vol. 90, no. 3, 1979, page 586, column 1, abstract no. 22327x, Columbus, Ohio, US; A. ONONPCHENKO et al.: "Carboxylic acids from olefins or olefin mixtures", connected with Chem. Subst. Index, page 2765CS, column 1, line 74

## Description

### BACKGROUND OF THE INVENTION

This invention relates to pure single isomers of neoalkyl carboxylic acids ("neoacids") and platinum complexes containing the single isomer neoacids. The use of liposomes incorporating these complexes in anti-tumor chemotherapy is also described. Particularly preferred for forming the liposomes are saturated phosphatidylcholines.

Cis-platinum (CDDP) is a highly effective drug in the treatment of several neoplastic diseases in humans (Loehrer et al. (1984) Ann. Int. Med., 100:704-713). However, its use is limited by severe systemic toxicity, particularly nephrotoxicity and neurotoxicity (Zwelling et al. Platinum Complexes. In: Pharmacologic Principles of Cancer Treatment (1982) Ed by B.A. Chabner, Saunders, Philadelphia, PA). In an attempt to modify the therapeutic index of CDDP, new derivatives have been synthesized during the last decade. However, the development of some promising analogues has been prevented by their limited hydrosolubility, which decreases their potential for clinical use (Burchenal et al. (1979) Cancer Treat. Rep. 63:1493-1497).

Liposomes are lipid vesicles which form spontaneously upon addition of an aqueous solution to a dry lipid film (Mayhew et al., In: Liposomes (1983) Marc J. Ostro, ed., Marcel Dekker, Inc., New York, N.Y.). Liposomes may be used as drug carriers of hydrophobic or hydrophilic drugs entrapped in their hydrophobic or hydrophilic compartments respectively. Multilamellar liposomes are particularly suited for carrying hydrophobic drugs since their hydrophobic space is larger than their hydrophilic compartment.

A liposome bilayer is composed of two lipid monolayers having a hydrophobic "tail" region and a hydrophilic "head" region. The structure of the membrane bilayer is such that the hydrophobic (nonpolar) "tails" of the lipid monolayers orient towards the center of the bilayer while the hydrophilic "heads" orient towards the aqueous phase.

The original liposome preparation of Bangham et al. (J. Mol. Biol., 1965, 12:238-252 involves suspending phospholipids in an organic solvent which is then evaporated to dryness leaving a phospholipid film on the reaction vessel. Next, an appropriate amount of aqueous phase is added, the mixture is allowed to "swell", and the resulting liposomes which consist of multilamellar vesicles (MLVs) are dispersed by mechanical means. This technique provides the basis for the development of the small sonicated unilamellar vesicles described by Papahadjopoulos et al. (Biochim. Biophys. Acta., 1968, 135:624-638).

Another class of liposomes that may be used are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 4,558,578 to Fountain, et al., and frozen and thawed multilamellar vesicles (FATMLV) wherein the vesicles are exposed to at least one freeze and thaw cycle; this procedure is described in Bally et al., PCT Publication No. 87/00043, January 15, 1987, entitled "Multilamellar Liposomes Having Improved Trapping Efficiencies". The methods and procedures described in these references are incorporated herein by reference.

In a liposomes-drug delivery system, a bioactive agent such as a drug is entrapped in or associated with the liposome and then administered to the patient to be treated. For example, see Rahman et al., U.S. Patent No. 3,993,754; Sears, U.S. Patent No. 4, 145,410; Papahadjopoulos et al., U.S. Patent No. 4,235,871; Schnieder, U.S. Patent No. 4,224,179; Lenk et al., U.S. Patent No. 4,522,803 and Fountain et al., U.S. Patent No. 4,588,578.

Liposomes have been previously used in vitro to deliver chemotherapeutic agents, (Mayhew et al., in: Liposomes (1983), Ostro, ed., Marcel Dekker, Inc., New York, N.Y.) and immunomodulators and anti-fungal agents in vitro (Mehta et al. (1984), Immunology V 51 pp 517-527, and in vivo in animals (Lopez-Berestein et al. (4)(1984) Clin. Exp. Metastasis V 2 pp 127-137 and Lopez-Berestein et al. (1983), J. Inf. Dis. V 147, pp. 937-945, J. Inf. Dis. V 151 pp. 704-710).

Recent studies show that liposomes can reduce certain types of drug-related toxicities such as doxorubicin cardiotoxicity (Forssen et al. (1981) Proc. Natl. Acad. Sci. V 78 pp 1873-1877, Olson et al. (1982), Eur. J. Cancer Clin. Oncol. V 18 pp. 167-176, Gabizon et al. (1982) Cancer Res. V 42 pp 4734-4739, Herman et al. (1983) Cancer Res. V 43 pp 5427-5432) and CDDP nephrotoxicity, (Freise et al. (1982), Arch. Inc. Pharmacodynamic Therapy V 258 pp 180-192) and may increase anti-tumor activity as a result of a slow release mechanism (Mayhew et al. (1978) Ann. N.Y. Acad. Sci. V 308, pp 371-386, Patel et al. (1984) Int. J. Cancer V 34 pp 717-723) a higher drug uptake by tumor cells or due to a more selective organ distribution (Gabizon et al. (1983) Cancer Res. V 43, pp 4730-4735 and Mayhew et al. (1983), Cancer Drug Deliv. V 1 pp 43-58). In U.S. Patent No. 4,330,534, N⁴-acylcytosine arabinoside incorporated into liposomes, for example, was found to be therapeutically effective when administered to tumor-bearing animals. In spite of these promising results, the clinical application of anti-tumor agents encapsulated in liposomes has been delayed, mainly due to formulation, drug stability and large scale production problems.

CDDP has been previously encapsulated in MLVs but with a very low encapsulation efficiency (7.4%) and poor stability (75% at 48 hours in 0.9% NaCl solution) (Freise et al. (1982) Arch. Int. Pharmacodynamic Therapy V 258 pp 180-192).

In U.S. Patent No. 4,256,652 are described certain platinum compounds comprising resolved stereoisomers of 1,2 diaminocyclohexane (DACH). The isomers utilized were cis-DACH, trans-RR-DACH and trans-SS-DACH. The platinum compounds described therein contained, in addition to a resolved DACH isomer, two hydrophilic platinum ligands such as bromide, iodide, nitrate, bromoacetate, sulfate or glucuronate.

In European Patent Application No. 83306726.7 certain platinum compounds are described which may comprise diaminocyclohexane (non-stereochemically resolved) and do comprise phosphatidyl groups having fatty acid substituents. These compounds are described as largely insoluble in plasma and preferably employed with lipid vesicle carriers. The platinum compound-phospholipid vesicles were preferably prepared by a sonic oscillation procedure which characteristically yields unilamellar vesicles.

Khokhar et al., PCT Publication No. WO87/02364, published April 23, 1987, discloses mixed isomers of neodecanoate platinum complexes of amines, which can be incorporated into liposomes; relevant portions of this reference are incorporated herein by reference.

Neoalkyl carboxylic acids are disclosed in:
DE-A-2044312 , EP-A-108489 , Weiske, T. and Schwartz, H., Chemische Berichte, 116,323-347 (1983), Souma, Y. and Sano, H., Bulletin of the Chemical Society of Japan, 49(11), 3296-3299 (1976), Chemical Abstracts, 99:177578q, & JP-A-58043869, SU 818654, & Chemical Abstracts, 95: 101020y, DE-B-2811886 and WO-A-87/02364.

### SUMMARY OF THE INVENTION

This invention relates to platinum complexes comprising pure single isomers of neoalkyl carboxylic acids. The use of liposomes incorporating these complexes and used in anti-tumor chemotherapy is also described. A particularly preferred liposomal formulation comprising a saturated phosphatidylcholine is described.

### DETAILED DESCRIPTION OF THE INVENTION

Previous attempts at synthesis of the above-described neoacids by hydrocarboxylation (the Koch reaction) have resulted invariably in mixed isomers of these compounds. Single isomer neoacids in metal complexes have heretofore not been described. There was no recognition of the need for such single isomers to overcome the problems associated with obtaining a pharmaceutically accepted, reproducable and characterizable complex. The methods of the present invention have enabled the preparation of single isomer neoacids of substantial purity. The term "substantial purity" shall be taken to mean a single isomer preparation of the neoacid compounds described hereinabove, in a purity of 99% or greater with respect to other contaminating isomers. In some cases, the single isomer is up to and greater than 99.9% pure as analyzed by gas chromotography-mass spectrophotometry.

### SINGLE ISOMER NEOACIDS

The single isomer neoacids (I) comprised in the complexes of this invention have the formula:

R₁ - CO₂ - H (I)

and have n carbon atoms.

R₁ has the general structure: 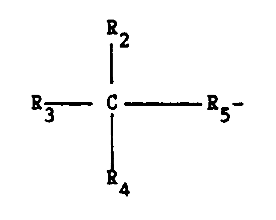
wherein R₂, R₃ and R₄ are each chosen from the groups comprising straight or branched chain alkyl or alkenyl, wherein R₅ is a straight or branched chain alkylene or alkenylene, wherein the total number of carbon atoms in the straight or branched chain alkyl or alkenyl in R₂, R₃ and R₄, and the straight or branched chain alkylene or alkenylene in R₅ is n-2, and wherein n is from about 6 to about 20 carbon atoms, preferably about 10 to about 20 carbon atoms, more preferably about 10 to about 11 carbon atoms, most preferably about 10 carbon atoms. R₂, R₃ and R₄ can each be about 1 to 16 carbon atoms. R₅ can be about 0 to 15 carbon atoms. In addition the carbon atoms of R₂, R₃, R₄ and R₅ can have substituents such as cycloalkyl or heterocyclic, preferably having about 3 to 10 carbon atoms; alkoxy, preferably having about 1 to 6 carbon atoms; aryl, preferably, but not limited to phenyl, naphthyl, or substituted phenyl or naphthyl where the substituent is preferably alkyl of about 1 to 6 carbon atoms.

Examples of the single isomer neoacids disclosed in the present invention and made by the methods of the invention include but are not limited to:
(1) 2,2-dimethyloctanoic acid
(2) 2-ethyl-2-methylheptanoic acid
(3) 2,2-diethylhexanoic acid
(4) 2,2-diethyl-4-methylpentanoic acid
(5) 2-ethyl-2,4,4-trimethylpentanoic acid
(6) 2-ethyl-2-methyloctanoic acid
(7) 2-ethyl-2-propylpentanoic acid

### PREPARATION OF SINGLE ISOMER NEOACIDS

The neoacids where R₅ is not present (i.e., R₅ contains 0 carbon atoms) can be made under anhydrous conditions, in an atmosphere that excludes oxygen, for example preferably under nitrogen gas, at a temperature of about 20-30°C by the addition of diisopropylamide, with sodium hydride (NaH) in mineral oil, and an organic solvent such as for example dry tetrahydrofuran (THF). Other solvents that can be employed are for example, ether solvents, such as diethyl ether, dioxane, or glymes and hexamethylphosphorictriamide. An alpha branched acid of 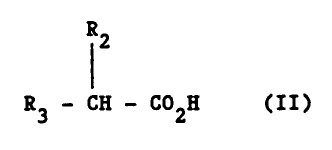
formula II such as isobutyric acid or 2-methylbutyric acid is then added and the temperature is allowed to increase to reflux for about 15 minutes at 50-60°C, forming the sodium salt of the branched acid. The solution is then cooled in an ice bath at about 0°C, and butyllithium in hexane added, making lithium diisopropylamide (LDA), at a temperature under about 10°C. The mixture is then heated at about 30-35°C for about 30 minutes, then cooled to about 0°C, forming the alpha lithiated isobutyric acid. A compound of formula (III) for example, bromopentane or bromohexane is then added, forming the neoacid (I). Other moieties that can be used as X are good nucleophilic leaving groups and include other halogens, tosylates, mesylates, brosylates, phosphates, or sulfates.

R₄ - X (III)

A precipitate of lithium bromide results when X is bromine and the mixture is then stirred for one-half hour in the ice bath, then heated at about 35°C for one hour, allowed to cool and sit overnight. Following acidification of the neoacid (I) solution with an acid such as hydrochloric acid, the neoacid (I) is extracted into an organic solvent such as ether, and purified by vacuum distillation to a substantially pure neoacid (I).

Compounds of formula I wherein R₅ contains one or more carbon atoms in the chain can be prepared by carbon chain addition methods known in the art.

### SINGLE ISOMER NEOACID PLATINUM (II) COMPLEXES

The single isomer neoacids of formula I can form complexes with metals, such as platinum. The single isomer neoacids can be complexed with platinum as part of four coördinate planar or six coördinate octahedral platinum complexes. In the case of platinum four coördinate planar complexes, the formula can be: 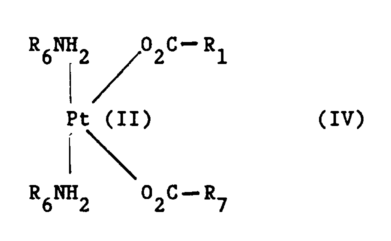
In this formula R₁ is as previously defined and R₇ can be the same or different R₁.

Alternatively, in this formula R₇ is a carboxylate bearing a hydrophobic radical function, most preferably neodecyl.

In the above-described complex the carboxylato of R₇ is preferably an alkylcarboxylato having between about 5 and 20 carbon atoms, an arylcarboxylato wherein aryl is phenyl, naphthyl, or an alkylphenyl wherein the alkyl phenyl has between about 12 and 16 carbon atoms. As used in the above description the term aryl is defined further as a function preferably having between about 6 and 14 carbon atoms.

R₆ is selected from the group consisting of hydrogen, an alkyl having from 1 to 20 carbon atoms including but not limited to groups such as methyl, ethyl, isopropyl, aryl, aralkyl, alkenyl, a cycloalkyl such as cyclohexyl, cycloalkenyl and a combination thereof. R₆ is preferably hydrogen, alkyl having between 1 and 20 carbon atoms, more preferably between 6 and 12 carbon atoms and most preferably between 2 and 6 carbon atoms or cycloalkyl having between 3 and 12 carbon atoms.

Additionally the two R₆ may be linked together, and are preferably selected from the group consisting of cycloalkyl-diamino functions having between about 3 and 10, more preferably between about 3 and 6 carbon atoms, and alkyl-diamino having between about 1 and 20, more preferably between about 2 and 12 carbon atoms, preferably ethyl. A preferred cycloalkyl-1,2-diamino component is 1,2-diaminocyclohexane, preferably in the trans-R,R-or trans-S,S-form, but also the racemate and cis forms R,S and S,R.

Examples of the single isomer neoacid trans-R,R-1,2-diaminocyclohexane platinum (II) complexes disclosed in the present invention, include but are not limited to the following:
(1) di-2,2-dimethyloctanoate-trans-R,R-1,2-diaminocyclohexane platinum (II) (2) di-2-ethyl-2-methylheptanoate-trans-R,R-1,2-diaminocyclohexane platinum (II)
(3) di-2,2-diethylhexanoate-trans-R,R-1,2-diaminocyclohexane platinum (II)
(4) di-2,2-diethyl-4-methylpentanoate-trans-R,R-1,2-diaminocyclohexane platinum (II)
(5) di-2-ethyl-2,4,4-trimethylpentanoate-trans-R,R-1,2-diaminocyclohexane platinum (II)
(6) di-2-ethyl-2-methyloctanoate-trans-R,R-1,2-diaminocyclohexane platinum (II)
(7) di-2-ethyl-2-propylpentanoate-trans-R,R-1,2-diaminocyclohexane platinum (II)
Other representative platinum (II) complexes are, for example, di-2,2-dimethylbutanoate-trans- R,R-1,2-diaminocyclohexane platinum(II), di-2-propylpentanoate-trans- R,R-1,2-diaminocyclohexane platinum(II), di-2-ethyl-2-methyl-pentanoate- trans-R,R-1,2-diaminocyclohexane platinum(II), di-2-ethyl-butanoate-trans- R,R-1,2-diaminocyclohexane platinum(II), di-2-ethyl-hexanoate-trans-R,R- 1,2-diaminocyclohexane platinum(II), di-2,2-diethylpentanoate-trans-R,R- 1,2-diaminocyclohexane platinum(II), di-2,2-dimethylpropanoate-trans-R,R- 1,2-diaminocyclohexane platinum(II), di-2-ethyl-2-methylbutanoate-trans-R,R-1,2-diaminocyclohexane platinum(II), di-2,2-diethylpentanoate-trans-R,R-1,2-diaminocyclohexane platinum(II), di-2,2-diethylnonanoate-trans-R,R-1,2-diaminocyclohexane platinum(II), di-octanoate-trans-R,R-1,2-diaminocyclohexane platinum(II), di-decanoate-trans-R,R-1,2-diaminocyclohexane platinum(II), di-2,2-diethylpentanoate-trans-R,R-1,2-diaminocyclohexane platinum(II), di-2,2-dimethylethanoate-trans-R,R-1,2-diaminocyclohexane platinum(II), and di-2,2-dimethyl-4-ethylhexanoate-trans-R,R-1,2-diaminocyclohexane platinum(II).

### PREPARATION OF PLATINUM (II) COMPLEXES WITH SINGLE ISOMER NEOACIDS

The compounds of formula IV can be prepared by any method known in the art such as those disclosed in Khokhar, et al., PCT Publication No. WO87/02364.

Alternatively, a tetracoördinate platinum (II) complex such as potassium tetrachloroplatinum (K₂PtCl₄) is dissolved in water or another polar solvent at a temperature of about between about 5-100°C, preferably 15-30°C, in a nitrogen atmosphere. The concentration of platinum complex to solvent is generally between about 10 and about 0.01 g/ml, preferably between about 1 and 0.05 g/ml. Alternatively, another platinum salt such as platinum sulfate or phosphate, or other platinum halogen can be used. This solution is filtered, for example, through a medium porosity scintered glass funnel.

Stoichiometric amounts of a solution of amines R₆NH₂, wherein the amines can be the same or different, are admixed with the aqueous solution of K₂PtCl₄. Thus, when R₆NH₂ is in the preferred method, a diaminocyclohexane (DACH) for example, trans-R,R-diaminocyclohexane is employed. The reaction is performed in an inert atmosphere such as nitrogen to exclude oxygen and carbon dioxide at a temperature of between about 15°C and 30°C with stirring for about 3-24 hours, preferably about 17 hours, to obtain the diamino onion product (V). A precipitate of the diamino anion product (V) results when carried out in water using K₂PtCl₄ as the starting platinum complex.

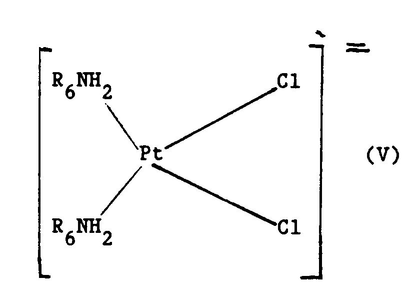

The filter cake is washed with water and acetone and dried under vacuum to a constant weight.

A suspension of silver sulfate is mixed in the dark at a temperature of between about 5-100°C, preferably at about 15-30°C, with deionized water, to form a suspension of about 10 to 0.1 grams silver sulfate per 100 ml of water, preferably about 1.5 g/100 ml, then stirred in the dark (in an amber vessel) for about 0.5 hours to about 24 hours at a temperature of about 10-60°C, preferably about 15-30°C, under an inert atmosphere, such as nitrogen. To this suspension is added the anionic amine platinum complex (V), and stirred for an additional about 5-48, preferably about 17 hours. The mixture is then filtered to remove the silver chloride salt, and the filter cake washed with water, preferably deionized water. The washings, containing the compound of formula (VI), were pooled with the filtrate and the filtrate was transferred to a dropping funnel.

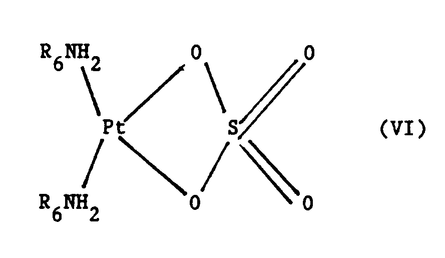

In order to react the single isomer neoacid (I) or acids of formula R₇-CO₂H with the platinum complex, salts of the carboxylic acids where the cation (M) can be sodium, potassium, ammonium, silver, barium, calcium, or other organic cation salts but preferably the sodium or silver salt are formed. For single isomer neoacids (I), the corresponding salts have formula (VII). 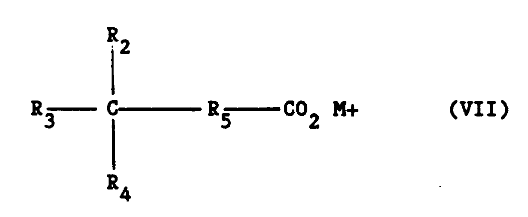
For other acids of formula R₇CO₂H, the corresponding salts are of formula R₇CO₂M. The carboxylic acids (I or R₇CO₂H) are dissolved or suspended in water, preferably deionized water, by forming a salt of the carboxylic acid at a concentration of between about 0.001 M and about 10 M compound. The preferred cation of the salt is sodium or potassium although any convenient cation can be employed. The salt is prepared by adding a base containing the cation to the carboxylic acid. For sodium hydroxide the preferred concentration is between about 0.1 M and about 10.0 M, preferably between about 2.5 M and 5.0 M. Additional solvent such as water is then added to obtain the desired concentration of the salts of the carboxylic in order to make the platinum complex (IV).

Alternatively, the sodium salt of the carboxylic may be made by dissolving the corresponding carboxylic acid in methanol and adding a stoichiometric amount of concentrated solution of sodium hydroxide. The pure sodium salt can then be obtained after evaporation of the solvent and precipitation of the product from acetone.

In order to prepare (IV), the carboxylic acid salt, in a concentration of about 50% w/v is placed in a flask. From a dropping funnel, the filtrate containing the sulfatoplatinum (VI) in about a 0.5-50% concentration is added about stoichiometrically to the stirred solution of carboxylic acid salt. The product (IV) begins to precipitate out of solution immediately upon addition of the sulfate group. This mixture is stirred gently for 1 hour to 5 days, preferably 1-17 hours, then extracted with an organic solvent such as for example, chloroform or methylene chloride. The organic layer is filtered into a round bottom flask and rotary evaporated to a residue at below about 50°C, preferably about 35°C.

The reaction product (IV) is extracted with a organic solvent in which (IV) is soluble, for example, halogenated solvent such as chloroform and is essentially insoluble in water.

The organic solvent is then removed, for example under reduced pressure to obtain (IV). (IV) can then be further purified by recystallization. For example, (IV) is taken up in the organic solvent and a polar organic solvent such as acetone. The resulting solution is then cooled to induce recrystallization of (IV), which is isolated by filtration.

The residue is then dissolved in an organic solvent such as but not limited to chloroform or methylene chloride, to which is added warm acetone. The two phases are mixed by swirling and the solution then placed in the cold room on ice for about 18 hours. The mixture is then filtered in the cold, for example, in a Buchner funnel through filter paper or a scintered glass filter and the filter cake washed with ice cold acetone and dried in vacuo to a constant weight, resulting in pure product as analyzed by HPLC.

For the product IV containing the single isomer neoacid (I) anions of the present inventions, (IV) is substantially free of other neoacid isomers. The term substantially free shall be taken to be at least about 95% of the single isomer, preferably at least about 97-99% of the single isomer, more preferably at least about 99% of the single isomer and most preferably at least about 99.9% of the single isomer as detected by gas chromatography or carbon magnetic resonance spectroscopy. The single isomer of formula I can include either racemic or optically active forms of the neoacid, when such optical isomers are possible.

### PLATINUM (VI) COMPLEXES WITH SINGLE ISOMER NEOACIDS

Yet another aspect of the instant invention is six-coördinate complexes containing the single isomer neoacids. Iproplatin, depicted below (VIII), is an example of a six coördinate platinum complex having utility as an antineoplastic agent. 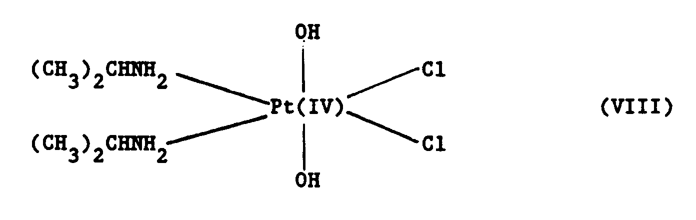
This compound can be derivatized to form the single isomer neoacid platinum complexes (IX): 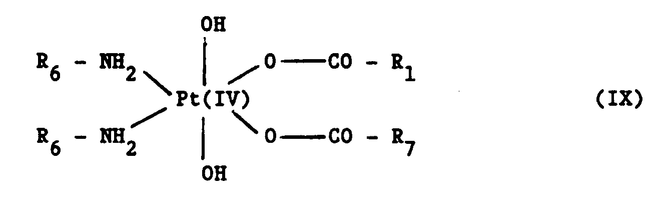
wherein R₁, R₆, and R₇ are as previously described. Other compounds of the present invention include those wherein other moieties are employed instead of the OH and R₆NH₂ groups in IX, such as halogen or carboxylic acid.

Such complexes can also be encapsulated in or associated with the liposomes of the invention according to the methods detailed hereinbelow.

### PREPARATION OF PLATINUM (IV) COMPLEXES WITH SINGLE ISOMER NEOACIDS

Compounds of formula IX, which include the corresponding six -coördinate complexes of (IV) can be prepared by oxidation of compounds such as V followed by addition of the carboxylic anion (VII) or R₇CO₂M).

These platinum IV compounds can also be prepared, for example, by the oxidations of IV, for example using the hydrogen peroxidation methods of Hydes et al., U.S. Patent 4,393,319, which is incorporated herein by reference.

The compounds of formula (IX) can be used therapeutically, for example, as an antineoplastic agent or as an antibacterial agent. The dosages of compounds of formula (IX) will generally be about those of VIII, i.e., cis-dichloro-trans-dihydroxy-bis (isopropylamine)-platinum (IV).

### PREPARATION OF LIPOSOMAL SINGLE ISOMER NEOACID PLATINUM COMPLEXES

As previously noted, liposomal complexes entrapping or associated with the compounds of formula IV are a further embodiment of the instant invention. Although any lipids can be used in the preparation of these liposomes, saturated phospholipids are preferred, such as for example the saturated phosphatidylcholines, for example dimyristoylphosphatidylcholine (DMPC), which can be used alone or in combination with other lipids such as saturated phosphatidyglycerols, for example, dimyristoylphosphatidylglycerol (DMPG). When DMPC and DMPG are used in combination, they are preferably in about a 7:3 DMPC:DMPG mole ratio. Other saturated lipids that can be used are for example, hydrogenated soy phosphatidylcholine (HSPC). In general, any lipid which is found to be soluble in an organic solvent such as t-butanol, and stable following liposome formation, and an evaporative process such as lyophilization, and reconstitution, is preferred. Stability can be determined by high performance liquid chromatography, thin layer chromotography, or other techniques. The liposomal formulation of metal complexes such as IV or IX is analyzed for the decomposition of the metal complex or any of the components of the liposome.

During preparation of the liposomes, organic solvents may be used to dissolve the lipids. Suitable organic solvents are those with a variety of polarities and dielectric properties, which solubilize the lipids, and include but are not limited to halogenated, aliphatic, cycloaliphatic, or aromatic-aliphatic hydrocarbons, such as benzene, chloroform, methylene chloride, or alcohols, such as methanol, ethanol, ¹²Freons, or tertiary butanol, and solvent mixtures such as benzene:methanol (70:30). As a result, solutions (mixtures in which the lipids and other components are uniformly distributed throughout) containing the lipids are formed. Solvents are generally chosen on the basis of their biocompatibility, low toxicity, and solubilization abilities. Further qualifications of the organic solvent used in the invention are that it should be evaporable, sublimable, or sufficiently miscible with water to enable its being removed, and that it should solubilize the metal complex such as IV or IX.

In the case of phosphatidylcholine, t-butanol is employed as the solvent. Where phosphatidylcholine and phosphatidylglycerol are used in for example, a 7:3 mole ratio, the solvents employed are for example, a mixture of t-butanol and methylene chloride. The lipid is dissolved in the solvent at a concentration of about 5 to about 75 mg/ml of t-butanol, preferably about 15 mg/ml of t-butanol. The platinum complex (IV) or (IX) is then admixed with the lipid and solvent, in amounts ranging from about 1:5 (drug:lipid) to about 1:30, preferably about 1:15, and is dissolved. The solution can then be sterilized, for exmple, by passage through a 0.2 um sterile filter, filled into 60 or 100 ml capacity (flint glass or amber) vials, fitted loosely with butyl rubber stoppers, and vacuum dried or lyophilized. The product can then, for example, be dehydrated for additional stability. One convenient method is by lyophilization.

For use, the lyophilized product is reconstituted in the vial by the addition of an aqueous solution such as water (Water for Injection), physiological saline solutions such as sodium chloride injection or other intravenous diluent such as for example dextrose injection, lactated Ringers injection to a concentration of 1 ml solution per mg drug, and hand shaken to suspend thereby forming a liposome dispersion. The reconstituted liposome particle size range is about 2 to about 15 um, preferably about 4.5 um. The particle size may be controlled such as by, for example, extrusion, milling or homogenization.

Important aspects of the present invention involve liposomes comprising fatty substances such as phospholipids, optionally cholesterol, and the four-coördinate platinum complexes described above, as well as the preparation and use of these liposomes. Liposomes of the present invention contain the platinum complex and the phospholipid in a preferred ratio between about 1 to 2 and about 1 to 30, a more preferred ratio being about 1 to 15.

Liposomes containing the platinum complexes described herein may be prepared from various amphipathic substances including natural or synthetic lipids such as phospholipids. The phospholipids usable to produce liposomes are numerous and not exhaustively listed herein since they are generally well known in the art. These phospholipids include but are not limited to: lecithin, phosphatidylethanolamine, lysolecithin, lysophosphatidylethanolamine, phosphatidylinositol, sphingomyelin, and the cerebrosides.

Preferred phospholipids of these liposomes including phosphatidylglycerol, phosphatidylcholine, sphingomyelin, phosphatidic acid or phosphatidylserine, the more preferred phospholipids being phosphatidylglycerol, phosphatidylcholine or a combination thereof. The most preferred phosphatidylglycerol is one consisting essentially of dimyristoylphosphatidylglycerol (DMPG) and the most preferred phosphatidylcholine is one consisting essentially of dimyristoylphosphatidylcholine (DMPC). When the liposomes of the present invention comprise dimyristoylphosphatidylglycerol and dimyristoylphosphatidylcholine they are preferably in a ratio between about 1:10 and 10:1, more preferably in a ratio of about 3:7. Alternatively, DMPC alone can be used. Other saturated or unsaturated lipids, for example, hydrogenated soy phosphatidylcholine (HSPC) or egg phosphatidylcholine (EPC) can also be used.

Cholesterol in minor proportions ranging from less than 1% to about 50% may be included with phospholipids and platinum complexes to produce liposomes of the present invention.

The liposomes of the present invention may be multilamellar, unilamellar or have an undefined lamellar construction. A pharmaceutical composition comprising the liposomes and neoacid compounds of the invention, together with a pharmaceutically acceptable carrier or dilutant may be used for the therapeutic, curative, remissive, retardive or prophylactic treatment of disease conditions such as cancer. The lipid may alternatively be present as micelles, or as a lipid emulsion.

Multilamellar liposomes are presently preferred since the platinum complexes of the present invention are substantially water - insoluble and they appear to be incorporated into hydrophobic region of the phospholipid bilayers of the liposome lamellae.

A focal point of the present invention involves a method of treating a host animal including a human afflicted with tumor cells sensitive to the presence of a platinum complex. This method comprises administering to the host an amount of the neoacid platinum complex described above or a liposome of the present invention comprising a phospholipid and a tumor cell-inhibiting effective amount of said neoacid platinum complex. The administering step is preferably parenteral and by intravenous, intraärterial, intramuscular, intralymphatic, intraperitoneal, subcutaneous, intrapleural or intrathecal injection or by topical application or oral dosage. Such administration is preferably repeated on a timed schedule, for example twice daily for a period of two weeks. The treatment may be maintained until tumor regression or disappearance has been achieved and may be used in conjunction with other forms of tumor therapy such as surgery or chemotherapy with different agents. As known to those skilled in the art, platinum-liposomes may be prepared as pellets, powders, or aerosols. These pellets or powders may be mixed with pharmaceutically acceptable solutions to form suspensions for parenteral administration. The aerosols can be administered intranasally.

The single isomer neoacid platinum - lipid compounds of the present invention can be used therapeutically in mammals, including man, in the treatment of infections or neoplasms which require the delivery of the drug in its bioactive form. Such conditions include but are not limited to disease states such as those that can be treated with the platinum compounds for the treatment of cancers in mammals such as humans.

The mode of administration of the preparation may determine the sites and cells in the organism to which the compounds will be delivered. The liposomes of the present invention can be administered alone but will generally be administered in admixture with a pharmaceutical carrier selected with regard to the intended route of administration and standard pharmaceutical practice. For parenteral administration, they can be used, for example, in the form of a sterile aqueous solution which may contain other solutes, for example, enough salts or glucose to make the solution isotonic. Other uses, depending upon the particular properties of the preparation, may be envisioned by those skilled in the art.

For administration to humans in the curative treatment of disease states responding to platinum therapy, the prescribing physician will ultimately determine the appropriate dosage for a given human subject, and this can be expected to vary according to the age, weight, and response of the individual as well as the nature, severity, and advancement of the patient's disease. The dosage of the drug in liposomal (or micellar, or emulsion, etc.) form will generally be about that employed for the free drug. In some cases, however, it may be necessary to administer dosages outside these limits. Oral or parenteral dosages of these platinum-liposomes between about 2.5 mg/kg body weight and 25 mg/kg body weight are contemplated as adequate in most conditions. The particular dosages, if a tumor-bearing human is being treated may vary in each case according to the condition of the patient, the type and extent of tumor, and particular platinum-liposome toxicity.

These anti-tumor methods may also be used to inhibit the metastatic spread of tumors such as reticulosarcoma. A preventative pretreatment with the platinum complexes or liposomes comprising these complexes may be used to preclude metastatic spread in a vaccination-like manner.

For the administeration of the platinum compounds of the present invention, a number of drug delivery systems can be employed, for example, liposomes, transdermal systems, gels, nasal sprays (aerosols), microsponges, implants, carrier molecules, monoclonal antibodies, osmotic pumps, and the like. In the present invention, the use of liposomes as the drug delivery system is preferred. Liposomes comprising phospholipids and platinum complexes of the present invention are useful in inhibiting both the growth and metastatic spread of tumors.

The amount of liposomal platinum included in the pharmaceutical composition and the dosage utilized in the method of treatment of the invention will vary depending in each case upon the conditions of the patients, the nature of the tumor undergoing treatment, anti-tumor activity of liposomal-platinum, the toxicity and solubility characteristics thereof, etc. Liposomal-platinum may also be administered in combination with other anti-tumor agents in a combined therapeutic regimen.

Topical administration of platinum-liposomes may involve pharmaceutical compositions such as suspensions, creams or ointments which may be obtained fully prepared or prepared from platinum-liposomes powders or pellets. Such topical administration may be near to sites of cancerous lesions such as the epithelium or mucosa for example.

Oral administrations of platinum-liposomes preferably involve encapsulation of platinum-liposome powder or pellets whereby the platinum-liposomes are protected from much gastric and intestinal digestive activity before release from the encapsulation.

When desired, platinum-liposomes may be prepared to contain, for example, other therapeutic agents for treatment of tumors or anti-oxidants to aid in liposome stabilization.

The following examples are presented to further illustrate preferred embodiments of the present invention and are not intended to limit the invention.

### EXAMPLE 1

### Preparation of 2,2-dimethyloctanoic acid

To a 2 L flask, attached to which is a source of dry, flowing nitrogen gas, was added 31 g (43 mL, 0.3 moles) of diisopropylamine, 13.5 g (0.3 moles) of 60% sodium hydride (NaH) in mineral oil, and 300 mL of tetrahydrofuran (THF). From a dropping funnel, was added 264 g (0.3 moles) of isobutyric acid at a temperature of 50-60°C over a 10 minute period, and refluxed for 15 minutes. The solution was then cooled in an ice bath at 0°C, and 187 mL of 2.5M butyllithium in hexane added through the stopper at a temperature under 10°C. The mixture was then heated at 30-35°C for 30 minutes, then cooled to 0°C, at which time 49.7 g (42.3 mL, 0.3 moles) of bromohexane was added from the dropper funnel. A precipitate of lithium bromide resulted and the mixture stirred for one half hour in the ice bath, then the mixture was heated at 35°C for one hour, then allowed to cool and sit overnight Deionized water (300 ML) was added, and the aqueous phase was set aside. To the organic phase was added 250 ml diethyl ether and 250 ml deionized water. The ether phase was discarded the aqueous phases pooled then extracted with 250 ml of diethyl ether. The phases were separated and discard the organic phase. The aqueous phase was acidified with 6N HCl to Congo Red. The producted was extracted with 2x250 ml of diethyl ether;t eh ether was extracted with 100 ml of saturated saline and dried over MgSO₄ overnight, filtered, evaporated to dryness and vacuum distilled yielding a pure product.

### EXAMPLE 2

### Preparation of di-2,2-dimethyloctanoate-trans-R,R-1,2-DACH Pt(II)

To a 12 L round bottom 3-neck flask equipped with an overhead stirrer, in the dark, was added 40.2 g (129 mmoles) of silver sulfate and 6 L of deionized water. The mixture was stirred in the dark for about 2 hours. To this stirred suspension was added a powder of 49 g (129 mmoles) of trans-R,R-diaminocyclohexane-dichloroplatinum (II). This mixture was stirred in the dark for 24 hours. The mixture was then filtered and and the filter cake washed with 3X50 mL of deionized water. The washings were pooled with the filtrate and the resulting solution containing DACH platinum sulfate was transferred to a dropping funnel.

In a second 12 L 3 neck flask equipped with a stirrer was added, under normal lighting conditions, 44.3 g (258 mmoles) of 2,2-dimethyloctanoic acid, followed by 51.6 mL (258 mmoles) of sodium hydroxide. Deionized water (500 ml) was then added to the flask to dissolve the sodium 2,2-dimethyloctanoate.

From the dropping funnel, the DACH platinum sulfate filtrate above was added dropwise to the stirred solution of sodium 2,2-dimethyloctanoate in the flask. This mixture was stirred for 17 hours, then extracted with 3X150 mL of chloroform. The chloroform layer was filtered into a 1 L round bottom flask and rotary evaporated to a residue at 35°C.

The residue was then dissolved in 100 mL of chloroform and 1000 mL of acetone was added. The two phases were mixed by swirling and the solution then placed in the cold room on ice for 18 hours. The mixture was then filtered in the cold in a Buchner funnel lined with filter paper and the filter cake washed with 3X 150 mL of ice cold acetone. The filter cake was dried in vacuo to a constant weight, resulting in pure product.

### EXAMPLE 3

### Preparation of 2-ethyl-2-methylheptanoic acid

The methods of Example 1 were followed using 2-methylbutyric acid in place of isobutyric acid, and bromopentane in place of bromohexane. The reaction yielded racemic mixtures of 99.9% pure neoacid.

### EXAMPLE 4

### Preparation of di-2-ethyl-2-methylheptanoate-trans-R,R-1,2-DACH Pt(II)

The methods of Example 2 were followed, employing 2-ethyl-2-methylheptanoic acid prepared by the methods of Example 3 instead of 2,2-dimethyloctanoic acid, thereby forming a sodium salt of 2-ethyl-2-methyl heptanoate rather than sodium 2,2-dimethyloctanoate. The 2-ethyl-2-methyl heptanoate was then reacted with the DACH sulfatoplatinum filtrate.

### EXAMPLE 5

### Preparation of 2,2-diethylhexanoic acid

The methods of Example 1 were followed using 2-ethylbutyric acid in place of isobutyric acid, and bromobutane in place of bromohexane.

### EXAMPLE 6

### Preparation of di-2,2-diethylhexanoate-trans-R,R-1,2-DACH Pt(II)

The methods of Example 2 were followed, employing 2-ethylbutyric acid prepared by the methods of Example 5 instead of 2,2-dimethyloctanoic acid, thereby forming a sodium salt of 2-ethyl butyric acid rather than sodium 2,2-dimethyloctanoate. The 2-ethylbutyric acid was then reacted with the DACH sulfatoplatinum filtrate.

### EXAMPLE 7

### Preparation of 2,2-diethyl-4-methylpentanoic acid

The methods of Example 1 were followed using 2-ethyl butyric acid in place of isobutyric acid, and 1-bromo-2-methylpropane in place of bromohexane.

### EXAMPLE 8

### Preparation of di- 2,2-diethyl-4-methylpentanoate-trans-R,R-1,2-DACH Pt(II)

The methods of Example 2 were followed, employing 2-2-diethyl-4-methylpentanoic acid prepared by the methods of Example 7 instead of 2,2-dimethyloctanoic acid, thereby forming a sodium salt of 2-2-diethyl-4-methylpentanoic acid rather than sodium 2,2-dimethyloctanoate. The 2-2-diethyl-4-methylpentanoic acid was then reacted with the DACH sulfatoplatinum filtrate.

### EXAMPLE 9

### Preparation of 2-ethyl-2,4,4-trimethylpentanoic acid

The methods of Example 1 were followed using 2-methylbutyric acid in place of isobutyric acid, and 1-bromo-2,2-dimethylpropane in place of bromohexane.

### EXAMPLE 10

### Preparation of di-2-ethyl-2,4,4-trimethylpentanoate-trans-R,R-1,2-DACH Pt(II)

The methods of Example 2 were followed, employing 2-ethyl-2,4,4-trimethylpentanoic acid prepared by the methods of Example 9 instead of 2,2-dimethyloctanoic acid, thereby forming a sodium salt of 2-ethyl-2,4,4-trimethylpentanoic acid rather than sodium 2,2-dimethyloctanoate. The 2-ethyl-2,4,4-trimethylpentanoic acid was then reacted with the DACH sulfatoplatinum filtrate.

### EXAMPLE 11

### Preparation of 2-ethyl-2,4,4-triethylpentanoic acid

The methods of Example 1 were followed using 2-ethylbutyric acid in place of isobutyric acid, and 1-bromo-2,2-dimethylpropane in place of bromohexane.

### EXAMPLE 12

### Preparation of di-2-ethyl-2,4,4-triethylpentanoate-trans-R,R-1,2-DACH Pt(II) acid

The methods of Example 2 were followed, employing 2-ethyl-2,4,4-triethylpentanoic acid prepared by the methods of Example 11 instead of 2,2-dimethyloctanoic acid, thereby forming a sodium salt of 2-ethyl-2,4,4-triethylpentanoic acid rather than sodium 2,2-dimethyloctanoate. The 2-ethyl-2,4,4-triethylpentanoic acid was then reacted with the DACH sulfatoplatinum filtrate.

### EXAMPLE 13

### Preparation of 2-ethyl-2-methyloctanoic acid

The methods of Example 1 were followed using 2-methylbutyric acid in place of isobutyric acid.

### EXAMPLE 14

### Preparation of di-2-ethyl-2-methyloctanoate-trans-R,R-1,2-DACH Pt(II)

The methods of Example 2 were followed, employing 2-ethyl-2-methyloctanoic acid prepared by the methods of Example 13 instead of 2,2-dimethyloctanoic acid, thereby forming a sodium salt of 2-ethyl-2-methyloctanoic acid rather than sodium 2,2-dimethyloctanoate. The 2-ethyl-2-methyloctanoic acid was then reacted with the DACH sulfatoplatinum filtrate.

### EXAMPLE 15

### Preparation of 2-ethyl-2-propylpentanoic acid

The methods of Example 1 were followed using 2-propylpentanoic acid in place of isobutyric acid.

### EXAMPLE 16

### Preparation of di-2-ethyl-2-propylpentanoate-trans-R,R-1,2-DACH Pt(II)

The methods of Example 2 were followed, employing 2-ethyl-2-propylpentanoic acid prepared by the methods of Example 15 instead of 2,2-dimethyloctanoic acid, thereby forming a sodium salt of 2-ethyl-2-propylpentanoic acid rather than sodium 2,2-dimethyloctanoate. The 2-ethyl-2-propylpentanoic acid was then reacted with the DACH sulfatoplatinum filtrate.

### EXAMPLE 17

### Preparation of the Liposomal-Neoacid DACH-Pt Complex

Dimyristoylphosphatidylcholine (DMPC) (450.0 mg) was dissolved in 10 ml of t-butanol. Di-2,2-dimethyloctanoate-trans-R,R-1,2-DACH Pt(II) was co-dissolved in the t-butanol at a ratio of 1 mg neoacid-Pt complex to 15 mg of DMPC (30 mg of neoacid-Pt). The solution was then filtered through a 0.2 um sterile filter, and filled into 60 ml flint glass vials and butyl rubber stoppers placed on top, but not fully seated, on the vials. The preparations were lyophilized according to Example 18 in a Stokes-Pennwalt lyophilizer, and stoppered under vacuum. The lyophilized product was reconstituted in 30 ml of 0.9% saline (1 ml saline per mg of drug), and the vial was hand shaken for about 2 minutes.

The above was repeated for bis (2,2-diethylhexanoate)-trans-R,R-1,2-DACH Pt(II) and bis (2,2-diethyl-4-methylpentanoate) -1,2-DACH Pt (II).

### EXAMPLE 18

### Lyophilization of the Liposomal-Neoacid DACH-Pt Complex

The liposomal platinum complex of Example 17 was lyophilized according to the following procedure. The lipid-neoacid-solvent was filled into a 60 ml capacity amber vial, and placed in a Stokes-Pennwalt Lyophilizer at room temperature. The shelf temperature was reduced to 10°C and held at that temperature for 0.5 hours. The shelf temperature was then ramped down to -45°C at a rate of 20°C per hour and held for 4 hours. The shelf temperature was then ramped up to -15°C at a rate of 10°C per hour and held for 2 hours. The shelf temperature was then ramped up to -5°C at a rate of 10°C per hour and held for 2 hours, then ramped up to 15°C at a rate of 10°C per hour and held for 2 hours. Finally, the shelf temperature was then ramped up to 30°C at a rate of 10°C per hour and held for 10 hours.

### EXAMPLE 19

The stability of the reconstituted DMPC-liposomal bis (2,2-dimethylocatanoate)-trans-R,R-1,2-DACH Pt(II) prepared in Example 17, was analyzed as follows. The product from 1 vial was reconstituted with 30 ml of 0.9% saline. This suspension was further diluted with saline in a 1:5 dilution in 0.9% saline, and 50 uL was injected into the sample port of a Waters or Rainin HPLC equipped with a chromegabond C8, 100A, 25 mm X 4.6.mm column. The running parameters were flow rate = 1.5 ml/min., run time =12 minutes, mobil phase = 90% methanol; elution time was about 4 minutes, detector was a Knaes Variable Wavelength; absorbance was determined at 211 nm. The area under the neoacid peak was observed for area at 24 hours following reconstitution, and compared to a sample of pure 2,2-dimethylocatanoate-trans-R,R-1,2-DACH Pt(II) prepared by the methods detailed below, similarly injected into the HPLC.

The product was also analyzed in the HPLC at 0, 1, 2, and 48 hours following reconstitution. In each case, peak areas were calculated and compared to the control profile of pure neoacid. Percentages of the peak area of the samples compared to peak area of the control were calculated.

The PC formulation was found to be over 90% stable after 24 hours following reconstitution (90% of the peak area of the control), having a particle size of an average of 4 um as measured by the Malvern Particle Sizer.

The above was repeated using 5.0% dextrose instead of 0.9% saline. The PC formulayion was similarly found to be over 90% stable after 24 hours following reconstitution.

The control was prepared as follows. A solution of 5 mg of the corresponding free platinum complex was prepared in 25 ml of methanol, and this solution injected (50 uL sample) into the HPLC. The area under the platinum complex peak was measured and compared to those platinum complex peaks derived from liposomal-platinum complex analyzed at 0, 1, 2, 24, and 48 hour timepoints.

## Claims (Claims for the following Contracting State(s): AT, BE, CH, DE, FR, GB, IT, LI, LU, NL, SE)

1. A substantially pure single isomer neoacid-platinum four-coördinate planar complex having the formula: 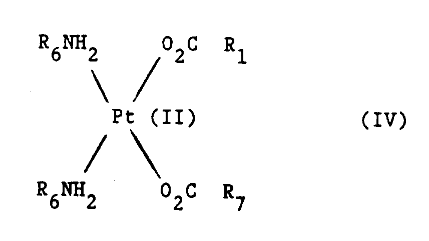 wherein R₁ has the general structure: 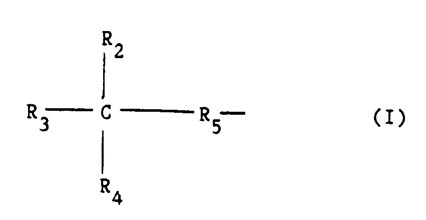 wherein R₂, R₃ and R₄ are each substituted or unsubstituted, straight or branched chain alkyl or alkenyl, and each has 1 to 16 carbon atoms;
wherein R₅ is a substituted or unsubstituted, straight or branched chain alkylene or alkenylene having 0 to 15 carbon atoms;
wherein the number of carbon atoms in the straight or branched chain alkyl or alkenyl in R₂, R₃ and R₄, and the straight or branched chained alkylene or alkenylene in R₅ is n-2;
wherein n is from 6 to 20;
wherein R₇ is the same or a different R₁, or R₇-COO- is an alkylcarboxylato having between 5 and 20 carbon atoms, an arylcarboxylato wherein aryl is phenyl, naphthyl, or an alkylphenyl wherein the alkyl phenyl has between 12 and 16 carbon atoms; and
wherein R₆ is hydrogen, alkyl having from 1 to 20 carbon atoms or cycloalkyl having between 3 and 10 carbon atoms; or
wherein the two R₆ are linked together to form a cycloalkyl-diamine having between 3 and 10 carbon atoms or an alkylciamine having between 1 and 20 carbon atoms.

2. The single isomer neoacid-platinum four coördinate planar complex of claim 1 wherein the cycloalkyl-diamine is 1,2-diaminocyclohexanyl.

3. The single isomer neoacid-platinum complex of claim 2 wherein the 1,2-diaminocyclohexanyl is in the trans-R,R or trans-S,S form, the racemate, or cis forms R,S or S,R.

4. The single isomer neoacid-platinum four coördinate planar complexes of claim 3 wherein the single isomer neoacid trans-R,R-1,2-diaminocyclohexane platinum complexes are di-2,2-dimethyloctanoate-trans-R,R-1,2-diaminocyclohexane platinum (II), di-2-ethyl-2-methylheptanoate-trans-R,R-1,2-diaminocyclohexane platinum (II), di-2,2-diethylhexanoate-trans-R,R-1,2-diaminocyclohexane platinum (II), di-2,2-diethyl-4-methylpentanoate-trans-R,R-1,2-diaminocyclo-hexane platinum (II), di-2-ethyl-2,4,4-trimethylpentanoate-trans-R,R-1,2-diaminocyclohexane platinum (II), di-2-ethyl-2-methyloctanoate-trans-R,R-1,2-diaminocyclohexane platinum (II), or di-2-ethyl-2-propylpentanoate-trans-R,R-1,2-diaminocyclohexane platinum (II).

5. A substantially pure single isomer neoacid platinum six-coördinate octahedral complex having the formula: 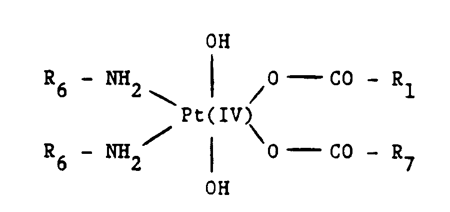 wherein R₁ has the general structure: 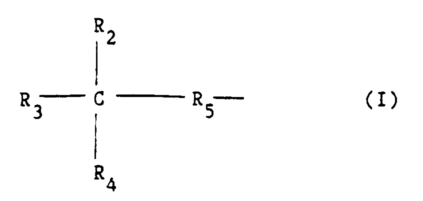 wherein R₂, R₃ and R₄ are each substituted or unsubstituted, straight or branched chain alkyl or alkenyl, and each have 1 to 16 carbon atoms;
wherein R₅ is a substituted or unsubstituted, straight or branched chain alkylene or alkenylene having 0 to 15 carbon atoms;
wherein the number of carbon atoms in the straight or branched chain alkyl or alkenyl in R₂, R₃ and R₄, and the straight or branched chained alkylene or alkenylene in R₅ is n-2;
wherein R₁ has n-1 carbon atoms;
wherein n is from 6 to 20 carbon atoms;
wherein R₇ is the same or a different R₁, or R₇-COO- is an alkylcarboxylato having between 5 and 20 carbon atoms, an arylcarboxylato wherein aryl is phenyl, naphthyl, or an alkylphenyl wherein the alkyl phenyl has between 12 and 16 carbon atoms; and
wherein R₆ is hydrogen, alkyl having from 1 to 20 carbon atoms or cycloalkyl having between 3 and 10 carbon atoms; or
wherein the two R₆ are linked together to form a cycloalkyl-diamine having between 3 and 10 carbon atoms or an alkyldiamine having between 1 and 20 carbon atoms.

6. A composition comprising a liposome and the single isomer neoacid-platinum four-coördinate planar complex of any one of claims 1 to 4.

7. The composition of claim 6 wherein the liposome comprises dimyristoylphosphatidylcholine.

8. The composition of claim 7 wherein the liposome additionally comprises dimyristoylphosphatidylglycerol.

9. A composition comprising a liposome and the single isomer neoacid-platinum six-coördinate octahedral complex of claim 5.

10. The composition of claim 9 wherein the liposome comprises dimyristoylphosphatidylcholine.

11. The composition of claim 10 wherein the liposome additionally comprises dimyristoylphosphatidylglycerol.

## Claims (Claims for the following Contracting State(s): ES, GR)

1. A method for the preparation of liposomes containing a substantially pure single isomer neoacid-platinum four-coordinate planar complex comprising:
(a) co-solublizing in an organic solvent a lipid and a compound having the formula: 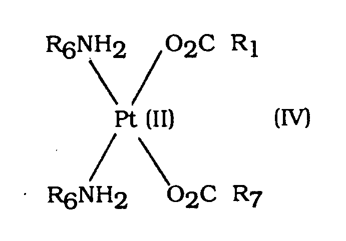 wherein R₁ has the general structure: 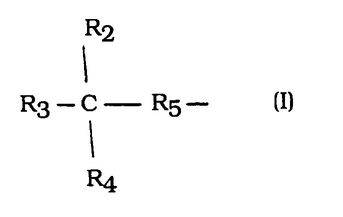 wherein R₂, R₃ and R₄ are each substituted or unsubstituted, straight or branched chain alkyl or alkenyl, and each has 1 to 16 carbon atoms;
wherein R₅ is a substituted or unsubstituted, straight or branched chain alkylene or alkenylene having 0 to 15 carbon atoms;
wherein the number of carbon atoms in the straight or branched chain alkyl or alkenyl in R₂, R₃ and R₄, and the straight or branched chained alkylene or alkenylene in R₅ is n-2;
wherein n is from 6 to 20;
wherein R₇ is the same or a different R₁, or R₇-COO- is an alkylcarboxylato having between 5 and 20 carbon atoms, an arylcarboxylato wherein aryl is phenyl, naphthyl, or an alkylphenyl wherein the alkyl phenyl has between 12 and 16 carbon atoms; and
wherein R₆ is hydrogen, alkyl having from 1 to 20 carbon atoms or cycloalkyl having between 3 and 10 carbon atoms; or
wherein the two R₆ are linked together to form a cycloalkyl-diamine having between 3 and 10 carbon atoms or an alkyldiamine having between 1 and 20 carbon atoms;
(b) removing the organic solvent; and
(c) hydrating the lipid to form liposomes.

2. The method of claim 1 wherein the cycloalkyl-diamine is 1,2-diaminocyclohexanyl.

3. The method of claim 2 wherein the 1,2-diamincyclohexanyl is in the trans-R,R-or trans-S,S-form, the racemate, or cis forms R,S or S,R.

4. The method of claim 3 wherein the single isomer neoacid trans-R,R-1,2-diaminocyclohexane platinum complexes are di-2,2-dimethyloctanoate- trans-R,R-1,2-diaminocyclohexane platinum (II), di-2-ethyl-2- methylheptanoate-trans-R,R-1,2-diaminocyclohexane platinum (II), di-2,2-diethylhexanoate-trans-R,R-1,2-diaminocyclohexane platinum (II), di-2,2-diethyl-4-methylpentanoate-trans-R,R-1,2-diaminocyclo- hexane platinum (II), di-2-ethyl-2,4,4-trimethylpentanoate- trans-R,R-1,2- diaminocyclohexane platinum (II), di-2-ethyl-2- methyloctanoate-trans-R,R-1,2-diaminocyclohexane platinum (II), or di-2-ethyl-2-propylpentanoate-trans-R,R- 1,2-diaminocyclohexane platinum (II).

5. The method of claim 1 wherein the lipid comprises dimyristoylphosphatidylcholine.

6. The method of claim 1 wherein the lipid additionally comprises dimyristoylphosphatidylglycerol.

7. A method for the preparation of a liposomes containing a substantially pure single isomer neoacid platinum six-coördinate octahedral complex comprising:
(a) co-solublizing in an organic solvent a lipid and a compound having the formula: 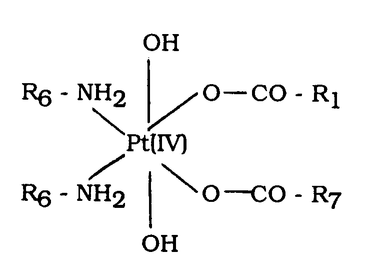 wherein R₁ has the general structure: 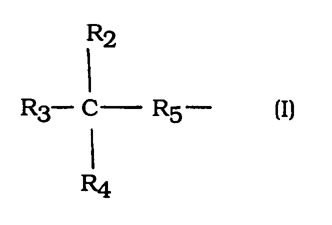 wherein R₂, R₃ and R₄ are each substituted or unsubstituted, straight or branched chain alkyl or alkenyl, and each has 1 to 16 carbon atoms;
wherein R₅ is substituted or unsubstituted, a straight or branched chain alkylene or alkenylene having 0 to 15 carbon atoms;
wherein the number of carbon atoms in the straight or branched chain alkyl or alkenyl in R₂, R₃ and R₄, and the straight or branched chained alkylene or alkenylene in R₅ is n-2;
wherein R₁ has n-1 carbon atoms;
wherein n is from 6 to about 20 carbon atoms;
wherein R₇ is the same or a different R₁, or substituted or unsubstituted, or R₇-COO- is an alkylcarboxylato having between 5 and 20 carbon atoms, an arylcarboxylato wherein aryl is phenyl, naphthyl, or an alkylphenyl wherein the alkyl phenyl has between 12 and 16 carbon atoms; and
wherein R₆ is hydrogen, alkyl having from 1 to 20 carbon atoms or cycloalkyl having between 3 and 10 carbon atoms; or
wherein the two R₆ are linked together to form a cycloalkyl-diamine having between 3 and 10 carbon atoms or an alkyldiamine having between 1 and 20 carbon atoms;
(b) removing the organic solvent; and
(c) hydrating the lipid to form liposomes.

8. The method of claim 7 wherein the lipid comprises dimyristoylphosphatidylcholine.

9. The method of claim 7 wherein the lipid additionally comprises dimyristoylphosphatidylglycerol.

## Patentansprüche (Patentansprüche für folgende(n) Vertragsstaat(en): AT, BE, CH, DE, FR, GB, IT, LI, LU, NL, SE)

1. Im wesentlichen reiner, planarer 4-fach-Koordinations-Einzelisomer-Neosäure-Platin-Komplex der Formel 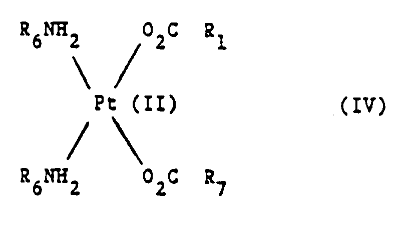 worin:
R₁ die allgemeine Struktur hat: 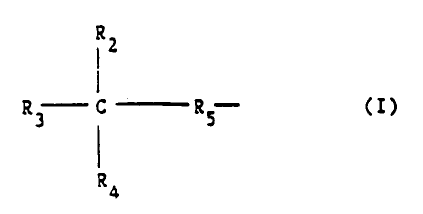
R₂, R₃ und R₄ jeweils stehen für substituiertes oder unsubstituiertes, geradkettiges oder verzweigtkettiges Alkyl oder Alkenyl mit jeweils 1 bis 16 Kohlenstoffatomen;
R₅ steht für ein substituiertes oder unsubstituiertes geradkettiges oder verzweigtkettiges Alkylen oder Alkenylen mit 0 bis 15 Kohlenstoffatomen;
wobei die Anzahl der Kohlenstoffatome in dem geradkettigen oder verzweigtkettigen Alkyl oder Alkenyl in R₂, R₃ und R₄ und in dem geradkettigen oder verzweigtkettigen Alkylen oder Alkenylen in R₅ n-2 beträgt;
n steht für eine Zahl von 6 bis 20;
R₇ gleich oder verschieden ist von R₁ oder R₇-COO- steht für ein Alkylcarboxylat mit 5 bis 20 Kohlenstoffatomen, ein Arylcarboxylat, worin Aryl Phenyl, Naphthyl oder ein Alkylphenyl darstellt, worin das Alkylphenyl 12 bis 16 Kohlenstoffatome aufweist; und
R₆ steht für Wasserstoff, Alkyl mit 1 bis 20 Kohlenstoffatomen oder Cycloalkyl mit 3 bis 10 Kohlenstoffatomen oder worin die zwei R₆-Reste miteinander verbunden sind unter Bildung eines Cycloalkyldiamins mit 3 bis 10 Kohlenstoffatomen oder eines Alkyldiamins mit 1 bis 20 Kohlenstoffatomen.

2. Planarer 4-fach-Koordinations-Einzelisomer-Neosäure-Platin-Komplex nach Anspruch 1, worin das Cycloalkyldiamin 1,2-Diaminocyclohexanyl ist.

3. Einzelisomer-Neosäure-Platin-Komplex nach Anspruch 2, worin das 1,2-Diaminocyclohexanyl in der trans-R,R- oder in der trans-S,S-Form, in Form des Racemats oder in der cis-R,S- oder cis-S,R-Form vorliegt.

4. Planare 4-fach-Koordinations-Einzelisomer-Neosäure-Platin-Komplexe nach Anspruch 3, worin die Einzelisomer-Neosäure-trans-R,R-1,2-Diaminocclohexan-Platin-Komplexe die folgenden sind Di-2,2-dimethyloctanoat-trans-R,R-1,2-Diaminocyclohexan-Platin(II), Di-2-ethyl-2-methylheptanoat-trans-R,R-1,2-Diaminocyclohexan-Platin(II), Di-2,2-diethylhexanoat-trans-R,R-1,2-Diaminocyclohexan-Platin(II), Di-2,2-diethyl-4-methylpentanoat-trans-R,R-1,2-Diaminocyclohexan- Platin(II), Di-2-ethyl-2,4,4-trimethylpentanoat-trans-R,R-1,2-Diaminocyclohexan-Platin(II), Di-2-ethyl-2-methyloctanoat-trans-R,R-1,2-Diaminocyclohexan-Platin(II) oder Di-2-ethyl-2-propylpentanoat-trans-R,R-1,2-Diaminocyclohexan-Platin(II).

5. Im wesentlichen reiner, octaedrischer 6-fach-Koordinations-Einzelisomer-Neosäure-Platin-Komplex der Formel 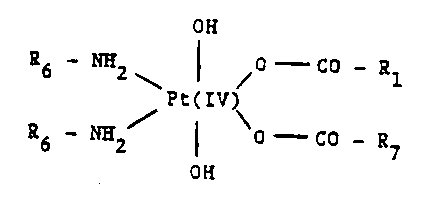 worin:
R₁ die allgemeine Struktur hat 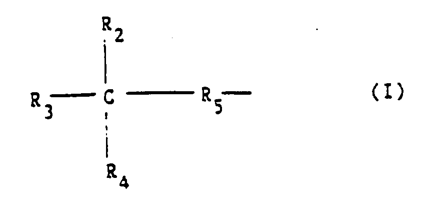
R₂, R₃ und R₄ jeweils stehen für substituiertes oder unsubstituiertes geradkettiges oder verzweigtkettiges Alkyl oder Alkenyl mit jeweils 1 bis 16 Kohlenstoffatomen;
R₅ steht für ein substituiertes oder unsubstituiertes, geradkettiges oder verzweigtkettiges Alkylen oder Alkenylen mit 0 bis 15 Kohlenstoffatomen;
wobei die Anzahl der Kohlenstoffatome in dem geradkettigen oder verzweigtkettigen Alkyl oder Alkenyl in R₂, R₃ und R₄ und in dem geradkettigen oder verzweigtkettigen Alkylen oder Alkenylen in R₅ n-2 beträgt;
R₁ n-1 Kohlenstoffatome aufweist;
n 6 bis 20 Kohlenstoffatome bedeutet;
R₇ gleich oder verschieden ist von R₁ oder R₇-COO- steht für ein Alkylcarboxylat mit 5 bis 20 Kohlenstoffatomen, ein Arylcarboxylat, worin das Aryl Phenyl, Naphthyl oder ein Alkylphenyl bedeutet, wobei das Alkylphenyl 12 bis 16 Kohlenstoffatome aufweist; und
R₆ steht für Wasserstoff, Alkyl mit 1 bis 20 Kohlenstoffatomen oder Cycloalkyl mit 3 bis 10 Kohlenstoffatomen oder worin die zwei R₆-Reste miteinander verbunden sind unter Bildung eines Cycloalkyldiamins mit 3 bis 10 Kohlenstoffatomen oder eines Alkyldiamins mit 1 bis 20 Kohlenstoffatomen.

6. Zusammensetzung, die umfaßt ein Liposom und den planaren, 4-fach-Koordinations-Einzelisomer-Neosäure-Platin-Komplex nach einem der Ansprüche 1 bis 4.

7. Zusammensetzung nach Anspruch 6, worin das Liposom Dimyristoylphosphatidylcholin umfaßt.

8. Zusammensetzung nach Anspruch 7, worin das Liposom außerdem Dimyristoylphosphatidylglycerin umfaßt.

9. Zusammensetzung, die umfaßt ein Liposom und den octaedrischen 6-fach-Koordinations-Einzelisomer-Neosäure-Platin-Komplex nach Anspruch 5.

10. Zusammensetzung nach Anspruch 9, worin das Liposom Dimyristoylphosphatidylcholin umfaßt.

11. Zusammensetzung nach Anspruch 10, worin das Liposom außerdem Dimyristoylphosphatidylglycerin umfaßt.

## Patentansprüche (Patentansprüche für folgende(n) Vertragsstaat(en): GR, ES)

1. Verfahren zur Herstellung von Liposomen, die einen im wesentlichen reinen planaren, 4-fach-Koordinations-Einzelisomer-Neosäure-Platin-Komplex enthalten, das umfaßt:
a) die gemeinsame Solubilisierung eines Lipids und einer Verbindung der Formel (IV) in einem organischen Lösungsmittel: 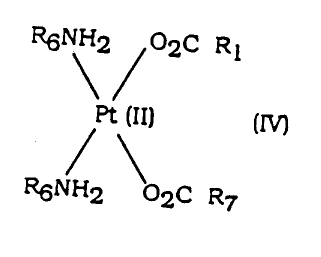 worin:
R₁ die allgemeine Struktur hat: 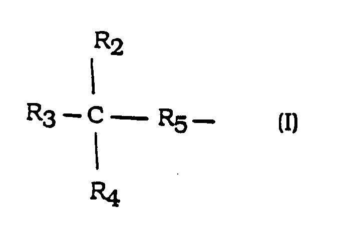
R₂, R₃ und R₄ jeweils stehen für substituiertes oder unsubstituiertes, geradkettiges oder verzweigtkettiges Alkyl oder Alkenyl mit jeweils 1 bis 16 Kohlenstoffatomen;
R₅ steht für ein substituiertes oder unsubstituiertes geradkettiges oder verzweigtkettiges Alkylen oder Alkenylen mit 0 bis 15 Kohlenstoffatomen;
wobei die Anzahl der Kohlenstoffatome in dem geradkettigen oder verzweigtkettigen Alkyl oder Alkenyl in R₂, R₃ und R₄ und in dem geradkettigen oder verzweigtkettigen Alkylen oder Alkenylen in R₅ n-2 beträgt;
n steht für eine Zahl von 6 bis 20;
R₇ gleich oder verschieden ist von R₁ oder R₇-COO- steht für ein Alkylcarboxylat mit 5 bis 20 Kohlenstoffatomen, ein Arylcarboxylat, worin Aryl Phenyl, Naphthyl oder ein Alkylphenyl darstellt, worin das Alkylphenyl 12 bis 16 Kohlenstoffatome aufweist; und
R₆ steht für Wasserstoff, Alkyl mit 1 bis 20 Kohlenstoffatomen oder Cycloalkyl mit 3 bis 10 Kohlenstoffatomen oder worin die zwei R₆-Reste miteinander verbunden sind unter Bildung eines Cycloalkyldiamins mit 3 bis 10 Kohlenstoffatomen oder eines Alkyldiamins mit 1 bis 20 Kohlenstoffatomen;
b) die Entfernung des organischen Lösungsmittels; und
c) die Hydratisierung des Lipids zur Bildung von Liposomen.

2. Verfahren nach Anspruch 1, worin das Cycloalkyldiamin 1,2-Diaminocyclohexanyl ist.

3. Verfahren nach Anspruch 2, worin das 1,2-Diaminocyclohexanyl in der trans-R,R- oder trans-S,S-Form, in Form des Racemats oder in der cis-R,S- oder cis-S,R-Form vorliegt.

4. Verfahren nach Anspruch 3, worin die Einzelisomer-Neosäure-trans-R,R-1,2-Diaminocyclohexan-Platin-Komplexe die folgenden sind Di-2,2-dimethyloctanoat-trans-R,R-1,2-Diaminocyclohexan-Platin(II), Di-2-ethyl-2-methylheptanoat-trans-R,R-1,2-Diaminocyclohexan-Platin(II), Di-2,2-diethylhexanoat-trans-R,R-1,2-Diaminocyclohexan-Platin(II), Di-2,2-diethyl-4-methylpentanoat-trans-R,R-1,2-Diaminocyclohexan-Platin(II), Di-2-ethyl-2,4,4-trimethylpentanoat-trans-R,R-1,2-Diaminocyclohexan-Platin(II), Di-2-ethyl-2-methyloctanoat-trans-R,R-1,2-Diaminocyclohexan-Platin(II) oder Di-2-ethyl-2-propylpentanoat-trans-R,R-1,2-Diaminocyclohexan-Platin(II).

5. Verfahren nach Anspruch 1, worin das Lipid Dimyristoylphosphatidylcholin umfaßt.

6. Verfahren nach Anspruch 1, worin das Lipid außerdem Dimyristoylphosphatidylglycerin umfaßt.

7. Verfahren zur Herstellung eines Liposoms, das einen im wesentlichen reinen, octaedrischen 6-fach-Koordinations-Einzelisomer-Neosäure-Platin-Komplex enthält, das umfaßt:
a) das gemeinsame Solubilisieren eines Lipids und einer Verbindung der folgenden Formel in einem organischen Lösungsmittel 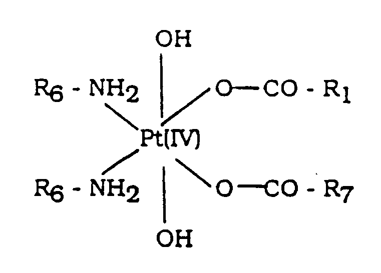 worin:
R₁ die allgemeine Struktur hat 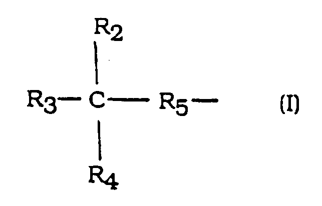
R₂, R₃ und R₄ jeweils stehen für substituiertes oder unsubstituiertes geradkettiges oder verzweigtkettiges Alkyl oder Alkenyl mit jeweils 1 bis 16 Kohlenstoffatomen;
R₅ steht für ein substituiertes oder unsubstituiertes, geradkettiges oder verzweigtkettiges Alkylen oder Alkenylen mit 0 bis 15 Kohlenstoffatomen;
wobei die Anzahl der Kohlenstoffatome in dem geradkettigen oder verzweigtkettigen Alkyl oder Alkenyl in R₂, R₃ und R₄ und in dem geradkettigen oder verzweigtkettigen Alkylen oder Alkenylen in R₅ n-2 beträgt;
R₁ n-1 Kohlenstoffatome aufweist;
n 6 bis 20 Kohlenstoffatome bedeutet;
R₇ gleich oder verschieden ist von R₁ oder R₇-COO- steht für ein Alkylcarboxylat mit 5 bis 20 Kohlenstoffatomen, ein Arylcarboxylat, worin das Aryl Phenyl, Naphthyl oder ein Alkylphenyl bedeutet, wobei das Alkylphenyl 12 bis 16 Kohlenstoffatome aufweist; und
R₆ steht für Wasserstoff, Alkyl mit 1 bis 20 Kohlenstoffatomen oder Cycloalkyl mit 3 bis 10 Kohlenstoffatomen oder worin die zwei R₆-Reste miteinander verbunden sind unter Bildung eines Cycloalkyldiamins mit 3 bis 10 Kohlenstoffatomen oder eines Alkyldiamins mit 1 bis 20 Kohlenstoffatomen;
b) das Entfernen des organischen Lösungsmittels; und
c) das Hydratisieren des Lipids zur Bildung von Liposomen.

8. Verfahren nach Anspruch 7, worin das Lipid Dimyristoylphosphatidylcholin umfaßt.

9. Verfahren nach Anspruch 7, worin das Lipid außerdem Dimyristoylphosphatidylglycerin umfaßt.

## Revendications (Revendications pour l'(les) Etat(s) contractant(s) suivant(s): AT, BE, CH, DE, FR, GB, IT, LI, LU, NL, SE)

1. Complexe plan tétracoordiné de platine et de néoacides isomères uniques à peu près purs, de formule : 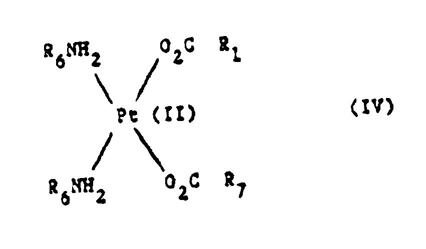 dans laquelle R₁ correspond à la structure générale : 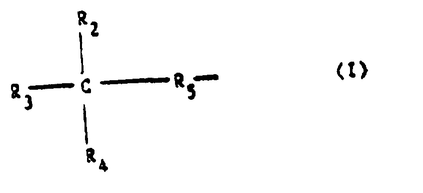 dans laquelle R₂, R₃ et R₄ représentent chacun un groupe alkyle ou alkényle à chaîne linéaire ou ramifiée, substitué ou non-substitué et comportant chacun de 1 à 16 atomes de carbone ;
dans laquelle R₅ représente un groupe alkylène ou alkénylène à chaîne linéaire ou ramifiée, substitué ou non substitué, comportant de 0 à 15 atomes de carbone ;
le nombre d'atomes de carbone dans le groupe alkyle ou alkényle, à chaîne linéaire ou ramifiée, de R₂, R₃ et R₄, et dans le groupe alkylène ou alkénylène à chaîne linéaire ou ramifiée, de R₅, étant égal à n-2 ;
n variant de 6 à 20 ;
dans laquelle R₇ est identique à ou différent de R₁, ou R₇-COO- représente un groupe alkylcarboxylato comportant de 5 à 20 atomes de carbone, un groupe arylcarboxylato dans lequel le groupe aryle est un groupe phényle, naphtyle ou un groupe alkylphényle, dans lequel le groupe alkylphényle comporte de 12 à 16 atomes de carbone ; et
dans laquelle R₆ représente un atome d'hydrogène, un groupe alkyle comportant de 1 à 20 atomes de carbone ou un groupe cycloalkyle comportant de 3 à 10 atomes de carbone ; ou
dans laquelle les deux groupes R₆ sont liés ensemble pour former un groupe dérivé de cycloalkyldiamine comportant de 3 à 10 atomes de carbone, ou un groupe dérivé d'alkyldiamine comportant de 1 à 20 atomes de carbone.

2. Complexe plan tétracoordiné de platine et de néoacides isomères uniques selon la revendication 1, dans lequel le groupe dérivé de cycloakyldiamine est un groupe 1,2-diaminocyclohexanyle.

3. Complexe de platine et de néoacides isomères uniques selon la revendication 2, dans lequel le groupe 1,2-diaminocyclohexamine est sous la forme trans-R,R ou trans-S,S, du racémate, ou sous la forme cis R,S ou S,R.

4. Complexes plans tétracoordinés de platine et de néoacides isomères uniques selon la revendication 3, dans lesquels les complexes de platine dérivés de trans-R,R-1,2-diaminocyclohexane et de néoacides isomères uniques, comprennent :
le di-2,2-diméthyloctanoate-trans-R,R-1,2-diaminocyclohexane platine (II),
le di-2-éthyl-2-méthyléthanoate-trans-R,R-1,2-diaminocyclo-hexane platine (II),
le di-2,2-diéthylhexanoate-trans-R,R-1,2-diaminocyclohexane platine (II),
le di-2,2-diéthyl-4-méthylpentanoate-trans-R,R-1,2-diamino-cyclohexane platine (II),
le di-2-éthyl-2,4,4-triméthylpentanoate-trans-R,R-1,2-diamino-cyclohexane platine (II),
le di-2-éthyl-2-méthyloctanoate-trans-R,R-1,2-diaminocyclo-hexane platine (II), ou
le di-2-éthyl-2-propylpentanoate-trans-R,R-1,2-diaminocyclo-hexane platine (II).

5. Complexe octaédrique hexacoordiné de platine et de néoacides isomères uniques à peu près purs, de formule : 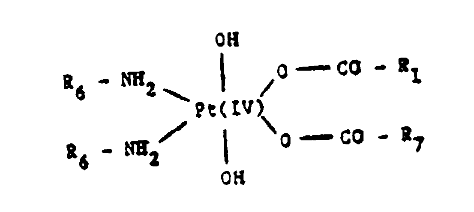 dans laquelle R₁ correspond à la structure générale : 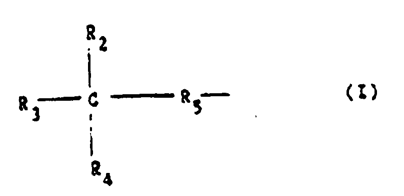 dans laquelle R₂, R₃ et R₄ représentent chacun un groupe alkyle ou alkényle à chaîne linéaire ou ramifiée, substitué ou non-substitué et comportant chacun de 1 à 16 atomes de carbone ;
dans laquelle R₅ représente un groupe alkylène ou alkénylène à chaîne linéaire ou ramifiée, substitué ou non substitué, comportant de 0 à 15 atomes de carbone ;
le nombre d'atomes de carbone dans le groupe alkyle ou alkényle, à chaîne linéaire ou ramifiée, de R₂, R₃ et R₄, et dans le groupe alkylène ou alkénylène à chaîne linéaire ou ramifiée, de R₅, étant égal à n-2 ;
dans laquelle R₁ contient n-1 atomes de carbone ;
n variant de 6 à 20 ;
dans laquelle R₇ est identique à, ou différent de R₁, ou R₇-COO- représente un groupe alkylcarboxylato comportant de 5 à 20 atomes de carbone, un groupe arylcarboxylato dans lequel le groupe aryle est un groupe phényle, naphtyle ou un groupe alkylphényle, dans lequel le groupe alkylphényle comporte de 12 à 16 atomes de carbone ; et
dans laquelle R₆ représente un atome d'hydrogène, un groupe alkyle comportant de 1 à 20 atomes de carbone ou un groupe cycloalkyle comportant de 3 à 10 atomes de carbone ; ou
dans laquelle les deux groupes R₆ sont liés ensemble pour former un groupe dérivé de cycloalkyldiamine comportant de 3 à 10 atomes de carbone, ou un groupe dérivé d'alkyldiamine comportant de 1 à 20 atomes de carbone.

6. Composition comprenant un liposome et le complexe plan tétracoordiné de platine et de néoacides isomères uniques selon l'une quelconque des revendications 1 à 4.

7. Composition selon la revendication 6, dans laquelle le liposome comprend de la dimyristoylphosphatidylcholine.

8. Composition selon la revendication 7, dans laquelle le liposome comprend en outre du dimyristoylphosphatidylglycérol.

9. Composition comprenant un liposome et le complexe octaédrique hexacoordiné de platine et de néoacides isomères uniques selon la revendication 5.

10. Composition selon la revendication 9, dans laquelle le liposome comprend de la dimyristoylphosphatidylcholine.

11. Composition selon la revendication 10, dans laquelle le liposome comprend en outre du dimyristoylphosphatidyl-glycérol.

## Revendications (Revendications pour l'(les) Etat(s) contractant(s) suivant(s): ES, GR)

1. Procédé de préparation de liposomes contenant un complexe plan tétracoordiné de platine et de néoacides isomères uniques à peu près purs, selon lequel :
(a) on dissout ensemble, dans un solvant organique, un lipide et un composé de formule : 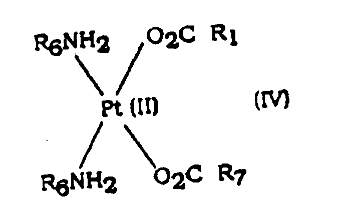 dans laquelle R₁ correspond à la structure générale : 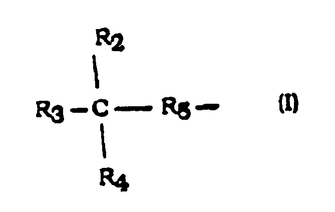 dans laquelle R₂, R₃ et R₄ représentent chacun un groupe alkyle ou alkényle à chaîne linéaire ou ramifiée, substitué ou non-substitué et comportant chacun de 1 à 16 atomes de carbone ;
dans laquelle R₅ représente un groupe alkylène ou alkénylène à chaîne linéaire ou ramifiée, substitué ou non substitué, comportant de 0 à 15 atomes de carbone ;
le nombre d'atomes de carbone dans le groupe alkyle ou alkényle, à chaîne linéaire ou ramifiée, de R₂, R₃ et R₄, et dans le groupe alkylène ou alkénylène à chaîne linéaire ou ramifiée, de R₅, étant égal à n-2 ;
n variant de 6 à 20 ;
dans laquelle R₇ est identique à ou différent de R₁, ou R₇-COO- représente un groupe alkylcarboxylato comportant de 5 à 20 atomes de carbone, un groupe arylcarboxylato dans lequel le groupe aryle est un groupe phényle, naphtyle ou un groupe alkylphényle, dans lequel le groupe alkylphényle comporte de 12 à 16 atomes de carbone ; et
dans laquelle R₆ représente un atome d'hydrogène, un groupe alkyle comportant de 1 à 20 atomes de carbone ou un groupe cycloalkyle comportant de 3 à 10 atomes de carbone ; ou
dans laquelle les deux groupes R₆ sont liés ensemble pour former un groupe dérivé de cycloalkyldiamine comportant de 3 à 10 atomes de carbone, ou un groupe dérivé d'alkyldiamine comportant de 1 à 20 atomes de carbone ;
(b) on élimine le solvant organique ; et
(c) on hydrate les lipides pour former des liposomes.

2. Procédé selon la revendication 1, dans lequel le groupe dérivé de cycloakyldiamine est un groupe 1,2-diaminocyclohexanyle.

3. Procédé selon la revendication 2, dans lequel le groupe 1,2-diaminocyclohexamine est sous la forme trans-R,R ou trans-S,S, du racémate, ou sous la forme cis R,S ou S,R.

4. Procédé selon la revendication 3, dans lequel les complexes de trans-R,R-1,2-diaminocyclohexane platine et de néoacides isomères uniques, comprennent :
le di-2,2-diméthyloctanoate-trans-R,R-1,2-diaminocyclohexane platine (II),
le di-2-éthyl-2-méthyléthanoate-trans-R,R-1,2-diaminocyclo-hexane platine (II),
le di-2,2-diéthylhexanoate-trans-R,R-1,2-diaminocyclohexane platine (II),
le di-2,2-diéthyl-4-méthylpentanoate-trans-R,R-1,2-diamino-cyclohexane platine (II),
le di-2-éthyl-2,4,4-triméthylpentanoate-trans-R,R-1,2-diamino-cyclohexane platine (II),
le di-2-éthyl-2-méthyloctanoate-trans-R,R-1,2-diaminocyclo-hexane platine (II), ou
le di-2-éthyl-2-propylpentanoate-trans-R,R-1,2-diaminocyclo-hexane platine (II).

5. Procédé selon la revendication 1, dans lequel le lipide comprend de la dimyristoylphosphatidylcholine.

6. Procédé selon la revendication 1, dans lequel le lipide comprend en outre du dimyristoylphosphatidylglycérol.

7. Procédé de préparation d'un liposome contenant un complexe octaédrique hexacoordiné de platine et de néoacides isomères uniques à peu près purs, selon lequel :
(a) on dissout ensemble dans un solvant organique, un lipide et un composé de formule : 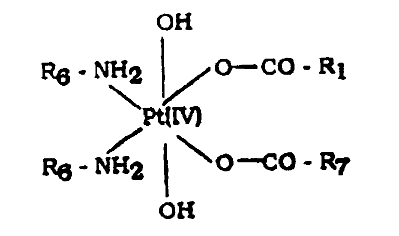 dans laquelle R₁ correspond à la structure générale : 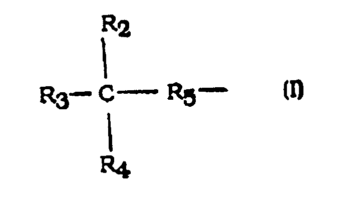 dans laquelle R₂, R₃ et R₄ représentent chacun un groupe alkyle ou alkényle à chaîne linéaire ou ramifiée, substitué ou non-substitué et comportant chacun de 1 à 16 atomes de carbone ;
dans laquelle R₅ représente un groupe alkylène ou alkénylène à chaîne linéaire ou ramifiée, substitué ou non substitué, comportant de 0 à 15 atomes de carbone ;
le nombre d'atomes de carbone dans le groupe alkyle ou alkényle, à chaîne linéaire ou ramifiée, de R₂, R₃ et R₄, et dans le groupe alkylène ou alkénylène à chaîne linéaire ou ramifiée, de R₅, étant égal à n-2 ;
dans laquelle R₁ contient n-1 atomes de carbone ;
n variant de 6 à 20 ;
dans laquelle R₇ est identique à, ou différent de R₁, ou R₇-COO- représente un groupe alkylcarboxylato comportant de 5 à 20 atomes de carbone, un groupe arylcarboxylato dans lequel le groupe aryle est un groupe phényle, naphtyle ou un groupe alkylphényle, dans lequel le groupe alkylphényle comporte de 12 à 16 atomes de carbone ; et
dans laquelle R₆ représente un atome d'hydrogène, un groupe alkyle comportant de 1 à 20 atomes de carbone ou un groupe cycloalkyle comportant de 3 à 10 atomes de carbone ; ou
dans laquelle les deux groupes R₆ sont liés ensemble pour former un groupe dérivé de cycloalkyldiamine comportant de 3 à 10 atomes de carbone, ou un groupe dérivé d'alkyldiamine comportant de 1 à 20 atomes de carbone ;
(b) on élimine le solvant organique ; et
(c) on hydrate le lipide pour former des liposomes.

8. Procédé selon la revendication 7, dans lequel le lipide comprend de la dimyristoylphosphatidylcholine.

9. Procédé selon la revendication 7, dans lequel le lipide comprend en outre du dimyristoylphosphatidylglycérol.
